# EUROPÄISCHE PATENTSCHRIFT

(11) **EP 3 233 140 B1**
(45) Veröffentlichungstag und Bekanntmachung des Hinweises auf die Patenterteilung: **21.05.2025**
(21) Anmeldenummer: 15820829.8
(22) Anmeldetag: 17.12.2015
(51) Int. Cl.: A61L 2/18, B67C 3/10

(54) **VERFAHREN UND VORRICHTUNG ZUM BEHANDELN EINES GEMISCHS AUS ENTLASTUNGSGAS UND FÜLLPRODUKTSCHAUM IN EINER GETRÄNKEABFÜLLANLAGE**
METHOD AND DEVICE FOR TREATING A MIXTURE OF EXPANSION GAS AND FILLING PRODUCT FOAM IN A BEVERAGE FILLING PLANT
PROCÉDÉ ET DISPOSITIF DE TRAITEMENT D'UN MÉLANGE COMPOSÉ D'UN GAZ DE DÉCHARGE ET D'UNE MOUSSE DE PRODUIT DE CHARGE DANS UNE INSTALLATION DE REMPLISSAGE DE BOISSON

(30) Priorität: 17.12.2014 DE 102014118815
(43) Veröffentlichungstag der Anmeldung: 25.10.2017
(73) Patentinhaber: Krones AG, 93073 Neutraubling (DE)
(72) Erfinder: KIENINGER, Stefan, 93073 Neutraubling (DE)
(74) Vertreter: Nordmeyer, Philipp Werner
(86) Internationale Anmeldenummer: PCT/EP2015/080160
(87) Internationale Veröffentlichungsnummer: WO 2016/097104

(56) Entgegenhaltungen:
- CA-A1- 2 313 891
- CN-A- 103 803 469
- CN-A- 103 803 469
- CN-U- 202 686 856
- CN-Y- 201 276 211
- CN-Y- 201 276 211
- DE-A1- 19 835 434
- DE-A1- 2 706 557
- DE-C- 312 484
- US-A- 2 792 029
- US-A- 2 792 029
- US-A- 4 009 118
- US-A- 4 166 801
- US-A- 4 338 218
- US-A1- 2014 235 147
- US-A1- 2017 043 279

## Beschreibung

### Technisches Gebiet

Die vorliegende Erfindung betrifft eine Vorrichtung und ein Verfahren zum Behandeln eines Gemischs aus Entlastungsgas und Füllproduktschaum in einer Getränkeabfüllanlage, bevorzugt zum Behandeln eines Gemischs aus CO₂ und Bierschaum.

### Stand der Technik

In Getränkeabfüllanlagen ist es bekannt, karbonisierte Getränke im Gegendruckverfahren abzufüllen, bei welchem der zu befüllende Behälter vor der eigentlichen Befüllung mit dem Füllprodukt mittels eines Spanngases vorgespannt wird und das karbonisierte Füllprodukt dann in den auf diese Weise vorgespannten, also unter Druck stehenden, zu befüllenden Behälter eingebracht wird. Durch das Aufbringen der Vorspannung auf den Behälter wird erreicht, dass das sich im Füllprodukt befindliche CO₂ während der Befüllung nicht übermäßig oder überhaupt nicht entbindet, so dass auf diese Weise ein übermäßiges Aufschäumen des Füllprodukts unterbunden werden kann. Damit kann der Füllvorgang schnell und auch exakt durchgeführt werden, da das Füllende beispielsweise beim Erreichen einer vorgegebenen Füllhöhe mit einer Füllstandssonde aufgrund der weitgehenden Abwesenheit von Füllproduktschaum genau bestimmt werden kann. Entsprechend kann ein karbonisiertes Füllprodukt auf diese Weise schnell und exakt abgefüllt werden.

Nach dem eigentlichen Befüllvorgang, wenn das Füllprodukt vollständig im dann befüllten Behälter aufgenommen ist, steht der Behälter immer noch unter Überdruck. Insbesondere im Kopfraum des Behälters liegt ein unter Druck stehendes Gas vor, welches im Wesentlichen ein Gemisch aus dem im karbonisierten Füllprodukt vorliegendem CO₂ und dem Vorspanngas ist. Wenn das Vorspanngas auch CO₂ ist, liegt entsprechend im Wesentlichen CO₂ unter Druck im Kopfraum vor.

Bevor der Behälter einer weiteren Verarbeitungsstufe zugeführt werden kann, beispielsweise einem Verschließer, muss er von dem jeweiligen Füllorgan gelöst werden. Davor muss entsprechend der Überdruck in dem Behälterkopfraum abgebaut werden. Hierzu ist es bekannt, den Überdruck kontrolliert mittels eines Entlastungsventils abzubauen. Hierbei wird durch das Öffnen des Entlastungsventils über eine Entlastungsleitung eine Fluidverbindung zwischen dem Kopfraum des Behälters und der Umgebung derart hergestellt, dass der sich im Kopfraum des Behälters befindliche Überdruck beim Öffnen des Entlastungsventils in die Umgebung abgebaut werden kann.

Bei karbonisierten Getränken kommt es dabei aber zu einem Aufschäumen des Füllprodukts, da durch das schnelle Entlasten des Kopfraumes ein spontanes Entbinden von CO₂ aus dem Füllprodukt stattfindet. Diese Aufschäumneigung ist bei der Abfüllung von Bier besonders ausgeprägt. Der auf diese Weise entstehende Schaum kann zusammen mit dem Entlastungsgas durch das Entlastungsventil in den Entlastungskanal eintreten und nach und nach durch diesen hindurch wandern. Das auf diese Weise im Entlastungskanal vorliegende Gemisch aus dem Entlastungsgas und dem Füllproduktschaum tritt am Ende des Entlastungskanals unkontrolliert aus. Am Ende des Entlastungskanals tropft der Füllproduktschaum üblicher Weise nach unten auf den Hallenboden beziehungsweise in einen Gully ab.

Die in der jeweiligen Abfüllhalle entstehenden Konzentrationen des Entlastungsgases, beispielsweise des CO₂, sind teilweise unerwünscht.

Die DE 312 484 C beschreibt eine Abfüllvorrichtung zum Füllen von Fässern und Flaschen mit schäumenden Flüssigkeiten. Weitere Abfüllvorrichtungen sind in der CN 103 803 469 A und CN 202 686 856 U beschrieben. Die US 2,792,029 A beschreibt einen Entschäumer für Milchabfüllmaschinen.

### Darstellung der Erfindung

Ausgehend von dem bekannten Stand der Technik ist es eine Aufgabe der vorliegenden Erfindung, ein verbessertes Verfahren zum Behandeln eines Gemischs aus Entlastungsgas und Füllproduktschaum in einer Getränkeabfüllanlage, sowie eine entsprechende Vorrichtung bereitzustellen.

Die Aufgabe wird durch ein Verfahren zum Behandeln eines Gemischs aus Entlastungsgas und Füllproduktschaum in einer Getränkeabfüllanlage mit den Merkmalen des Anspruchs 1 gelöst.

Vorteilhafte Weiterbildungen des Verfahrens ergeben sich aus den Unteransprüchen sowie der vorliegenden Beschreibung und den Figuren.

Entsprechend wird ein Verfahren zum Behandeln eines Gemischs aus Entlastungsgas und Füllproduktschaum in einer Getränkeabfüllanlage vorgeschlagen, umfassend die Schritte des Einführens des Gemisches aus Entlastungsgas und Füllproduktschaum in einen geschlossenen Abscheidebehälter und des Absaugens des Entlastungsgases aus dem geschlossenen Abscheidebehälter.

Dadurch, dass das Gemisch aus dem Entlastungsgas und dem Füllproduktschaum in einen geschlossenen Abscheidebehälter eingebracht wird und das Entlastungsgas aus dem Abscheidebehälter abgezogen wird, wird zum einen erreicht, dass eine Belastung der Umgebung der Getränkeabfüllanlage mit dem Entlastungsgas vermieden wird und entsprechend eine gesundheitliche Beeinträchtigung der Mitarbeiter an der Füllanlage durch das Entlastungsgas verringert beziehungsweise vermieden werden kann. Zum anderen kann eine Belastung der Umgebung um die Getränkeabfüllanlage herum mit Füllproduktschaum durch die geschlossene Ausführung des Abscheidebehälters vermieden werden.

Entsprechend ergibt sich durch das Verfahren eine geringere Belastung der direkten Umgebung um die Getränkeabfüllanlage herum - sowohl durch Entlastungsgas als auch durch Füllproduktschaum - wodurch eine gesundheitliche Beeinträchtigung der Mitarbeiter reduziert werden kann und insgesamt eine hygienischere und ästhetischere Abfüllung ermöglicht wird.

Erfindungsgemäß wird auf das in den geschlossenen Abscheidebehälter eingeführte Gemisch aus Entlastungsgas und Füllproduktschaum ein flüssiges Medium zur Beregnung aufgebracht, um ein Niederschlagen und Verflüssigen des Füllproduktschaums in dem Abscheidebehälter zu erreichen und entsprechend ein Absaugen des Füllproduktschaums aus dem geschlossenen Abscheidebehälter in die Absaugung für das Entlastungsgas zu verhindern.

In einer besonders bevorzugten Ausbildung des Verfahrens wird zur Beregnung des Gemischs aus Entlastungsgas und Füllproduktschaum das flüssige Medium aus dem geschlossenen Abscheidebehälter, bevorzugt aus einem unterem Bereich des geschlossenen Abscheidebehälters, entnommen und das flüssige Medium bevorzugt in einem Kreislauf geführt.

Auf diese Weise und insbesondere durch die Kreislaufführung kann auf ökonomisch und ökologisch sinnvolle Art und Weise eine ständige, konstante, periodische oder bedarfsweise Beregnung des Gemisches aus dem Entlastungsgas und dem Füllproduktschaum erreicht werden, derart, dass ein zuverlässiges Niederhalten des Füllproduktschaumniveaus innerhalb des geschlossenen Abscheidebehälters erreicht wird. Durch die Führung der Flüssigkeit im Kreislauf wird weiterhin eine ökonomische Ausbildung dahingehend erreicht, dass ein stetes Zuführen von Frischwasser nicht notwendig ist, sondern das flüssige Medium zur Beregnung wiederverwendet werden kann.

Bevorzugt wird im unteren Bereich, bevorzugt in einem Sumpf, des geschlossenen Abscheidebehälters ein flüssiges Medium bereitgestellt, welches bevorzugt zur Beregnung des Gemischs aus Entlastungsgas und Füllproduktschaum, so wie oben beschrieben, entnommen wird.

Das flüssige Medium kann dabei ursprünglich aus bereitgestelltem Frischwasser bestehen und wird mit dem Start des Verfahrens zur Behandlung des Gemisches aus Entlastungsgas und Füllproduktschaum immer weiter mit Komponenten des Füllprodukts aus dem niedergeschlagenen und verflüssigten Füllproduktschaum vermischt.

Bevorzugt wird das flüssige Medium aus dem geschlossenen Abscheidebehälter mit dem durch die Beregnung verflüssigten Füllproduktschaum aus dem geschlossenen Abscheidebehälter kontinuierlich, periodisch oder bedarfsweise abgeführt. Die bedarfsweise Abführung kann beispielsweise beim Eintritt einer zeitlichen Bedingung oder beim Erreichen einer Füllhöhe des flüssigen Mediums in dem geschlossenen Abscheidebehälter ausgelöst werden, derart, dass ab dem Erreichen einer vorgegebenen Füllhöhe die entsprechend überflüssige Flüssigkeit aus dem geschlossenen Abscheidebehälter abgezogen wird. Die abführte Flüssigkeit kann beispielsweise einer Abwasserleitung zugeführt werden.

Um ein effizientes Abscheiden des Füllproduktschaums beziehungsweise der Füllproduktkomponenten aus dem Gemisch aus dem Entlastungsgas und dem Füllproduktschaum bereits beim Eintritt in den geschlossenen Abscheidebehälter zu erreichen, findet das Einströmen des Gemisches aus dem Entlastungsgas und dem Füllproduktschaum bevorzugt tangential in einen zylinderförmigen oder konusförmigen Abscheidebehälter so statt, dass aufgrund der wirkenden Zentrifugalkräfte bereits ein erstes Anlegen des Füllproduktschaumes an die Innenwand des geschlossenen Abscheidebehälters und damit ein Trennen von Entlastungsgas und Füllproduktschaum stattfindet.

Bevorzugt wird im geschlossenen Abscheidebehälter das Entlastungsgas oberhalb der Beregnung abgesaugt, so dass ein Ansaugen des Füllproduktschaums durch die Absaugung vermieden werden kann.

In einer weiteren bevorzugten Ausbildung wird zur Reinigung ein Reinigungs- und/oder Sterilisationsmedium in den geschlossenen Abscheidebehälter eingebracht und wieder aus diesem abgeleitet, wobei das Reinigungs- und/oder Sterilisationsmedium bevorzugt auf die Innenwand des geschlossenen Abscheidebehälters aufgesprüht wird und im unteren Bereich des geschlossenen Abscheidebehälters ableitet wird. So kann eine hygienische Reinigung des Abscheidebehälters erreicht werden. Ein CIP-Medium kann weiterhin auch durch den Entlastungskanal zum Füllventil beziehungsweise von dem Füllventil durch den Entlastungskanal in den geschlossenen Abscheidebehälter geführt werden.

Unter einem geschlossenen Abscheidebehälter wird hier verstanden, dass dieser keine direkte Verbindung mit der Umgebung aufweist, sondern ein Zuführen von Medien und Fluiden in den geschlossenen Abscheidebehälter sowie ein Abführen von Medien und Fluiden aus dem geschlossenen Abscheidebehälter nur kontrolliert über entsprechende Rohrleitungen stattfindet.

Dabei wird in dem hier vorgestellten Verfahren zum einen erreicht, dass das Gemisch aus dem Entlastungsgas und dem Füllproduktschaum kontrolliert innerhalb eines geschlossenen Systems geführt wird und zum anderen das Entlastungsgas ebenfalls kontrolliert innerhalb eines geschlossenen Ablaufes geführt wird, so dass das Entlastungsgas beispielsweise kontrolliert über einen Kamin abgeleitet werden kann, ohne dass ein Eintragen von Entlastungsgas in die jeweilige Abfüllhalle stattfindet.

Die oben gestellte Aufgabe wird weiterhin durch eine Vorrichtung zum Behandeln eines Gemischs aus Entlastungsgas und Füllproduktschaum in einer Getränkeabfüllanlage mit den Merkmalen des Anspruchs 8 gelöst. Vorteilhafte Weiterbildungen ergeben sich aus den Unteransprüchen.

Entsprechend wird eine Vorrichtung zum Behandeln eines Gemischs aus Entlastungsgas und Füllproduktschaum in einer Getränkeabfüllanlage vorgeschlagen, welche einen Abscheidebehälter umfasst, in welchen eine das Gemisch aus Entlastungsgas und Füllproduktschaum führende Entlastungsleitung einmündet, wobei aus dem Abscheidebehälter über eine Absaugung das Entlastungsgas abgesaugt wird. Erfindungsgemäß ist der Abscheidebehälter geschlossen.

Damit ergeben sich die gleichen vorteilhaften Wirkungen, die bereits weiter oben zum Verfahren beschrieben wurden.

Erfindungsgemäß ist eine Beregnungsdüse in dem geschlossenen Abscheidebehälter vorgesehen, welche ein flüssiges Medium auf das im geschlossenen Abscheidebehälter vorliegende Gemisch aus Entlastungsgas und Füllproduktschaum aufbringt. Durch die Beregnung kann ein Niederhalten und Zerschlagen beziehungsweise Verflüssigen des Füllproduktschaums in dem geschlossenen Abscheidebehälter erreicht werden.

Weiter bevorzugt ist in einem unteren Bereich des geschlossenen Abscheidebehälters ein Ablauf, bevorzugt ein an einem Sumpf des geschlossenen Abscheidebehälters vorgesehener Ablauf, vorgesehen und der Ablauf steht mit der Beregnungsdüse in Fluidkommunikation, so dass das flüssige Medium bevorzugt im Kreislauf von dem unteren Bereich des geschlossenen Abscheidebehälters zu der Beregnungsdüse gepumpt werden kann.

Bevorzugt ist auch, eine Absaugung zum Absaugen des Entlastungsgases in Fluidkommunikation mit dem geschlossenen Abscheidebehälter vorzusehen und die Absaugung bevorzugt oberhalb der Beregnungsdüse aus dem geschlossenen Abscheidebehälter zu führen.

Mit Vorteil ist der geschlossene Abscheidebehälter im Wesentlichen zylinderförmig und/oder konisch ausgebildet und die Entlastungsleitung ist tangential in den geschlossenen Abscheidebehälter eingeführt.

In einer weiteren bevorzugten Weiterbildung ist in dem geschlossenen Abscheidebehälter eine CIP-Düse zum Beaufschlagen der Innenwand des geschlossenen Abscheidebehälters vorgesehen und der geschlossene Abscheidebehälter steht in einem unteren Bereich in Fluidkommunikation mit einer CIP-Rückleitung.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine schematische Darstellung einer Getränkeabfüllanlage mit einem Füllerkarussell, einem Ventilknoten sowie einer Vorrichtung zum Behandeln eines Gemischs aus einem Entlastungsgas und Füllproduktschaum mit einem geschlossenen Abscheidebehälter;
- Figur 2: eine schematische Seitenansicht auf eine Vorrichtung zum Behandeln eines Gemischs aus einem Entlastungsgas und Füllproduktschaum; und
- Figur 3: eine schematische perspektivische Darstellung der Vorrichtung aus Figur 2.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

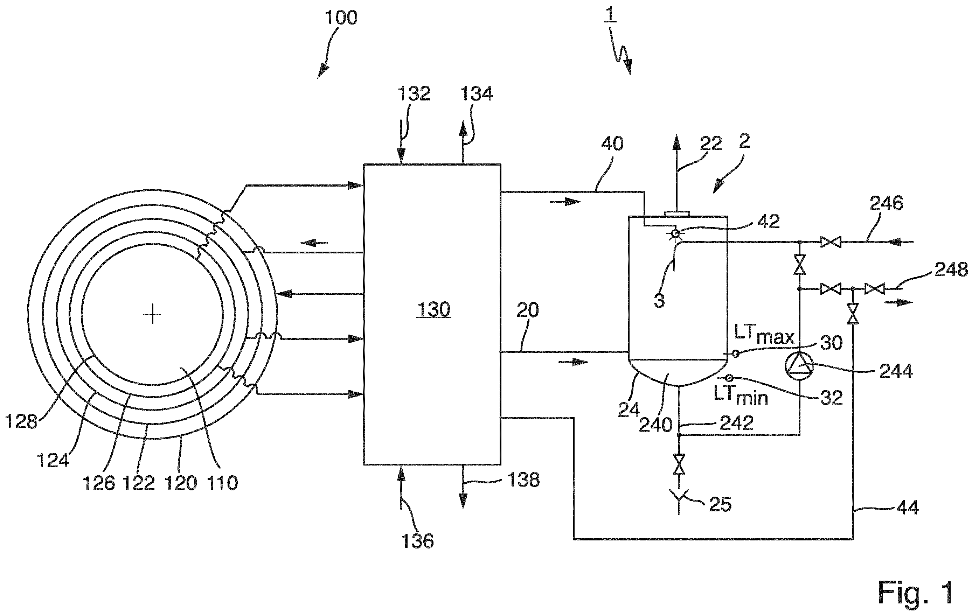

In Figur 1 ist schematisch eine Getränkeabfüllanlage 100 gezeigt, welche ein schematisch angedeutetes Füllerkarussell 110 aufweist, das ebenfalls schematisch angedeutete Mediendrehverteilerkanäle 120 bis 128 aufweist, welche zum Übergeben von Medien von dem stehenden Teil der Getränkeabfüllanlage auf das sich drehende Füllerkarussell 110 vorgesehen sind.

Der äußere Mediendrehverteilerkanal 120 ist zum Zuführen von Füllprodukt und - während der Reinigung und Sterilisierung der Getränkeabfüllanlage 100 - von Reinigungs- und Sterilisationsmedium auf das Füllerkarussell 110 vorgesehen. Der zweite Mediendrehverteilerkanal 122 ist zum Zuführen von CO₂ als Spülgas beziehungsweise als Spanngas vorgesehen. Der dritte Mediendrehverteilerkanal 124 ist als Entlastungskanal zur Entlastung des Kopfraums des jeweils befüllten Behälters nach der Befüllung vorgesehen und transportiert entsprechend ein Gemisch aus dem Entlastungsgas CO₂ und einem mit dem Entlastungsgas mitgerissenen Füllproduktschaum.

Ein weiterer Mediendrehverteilerkanal 126 ist zum Bereitstellen eines Vakuums beziehungsweise eines Unterdrucks vorgesehen, welches zum Schalten des Füllventils und/oder zum Evakuieren des zu befüllenden Behälters vor dem Füllen vorgesehen ist. Ein weiterer Mediendrehverteilerkanal 128 ist vorgesehen, welcher als CIP-Rücklauf für eine CIP-Reinigung oder Sterilisierung vorgesehen ist.

Das Zuführen beziehungsweise Abführen sowie das Steuern der Zufuhr der jeweiligen Medien auf den Mediendrehverteiler und die entsprechende Mediendrehverteilerkanäle 120 bis 128 findet über einen Ventilknoten 130 statt, welcher im gezeigten Ausführungsbeispiel im stehenden Teil der Getränkeabfüllanlage 100 vorgesehen ist und welcher die Mediendrehverteilerkanäle 120 bis 128 mit den jeweiligen Medien gesteuert beaufschlagen kann.

In den Ventilknoten 130 fließt über einen Füllproduktzulauf 132 das Füllprodukt beziehungsweise - während der Reinigung oder Sterilisation - ein Reinigungs- oder Sterilisationsmedium als CIP-Medium in den Ventilknoten 130 hinein. Ein CIP-Rücklauf 134 ist vorgesehen, mittels dessen das CIP-Medium entsprechend wieder an eine CIP-Station zum Aufbereiten beziehungsweise Austauschen des CIP-Mediums während der Reinigung oder Sterilisierung geleitet werden kann. Weiterhin wird dem Ventilknoten 130 gasförmiges CO₂ über einen CO₂-Zulauf 136 zugeführt, um dieses Gas entsprechend als Vorspann- und Spülmedium in dem zu befüllenden Behälter zu verwenden. Weiterhin ist ein Vakuumanschluss 138 vorgesehen, mittels welchem dem Ventilknoten 130 ein Vakuum schaltbar bereitgestellt werden kann.

Eine Vorrichtung 1 zum Behandeln eines Gemischs aus Entlastungsgas und Füllproduktschaum ist ebenfalls in der Getränkeabfüllanlage 100 vorgesehen. Dabei wird das Gemisch aus Entlastungsgas und Füllproduktschaum der Vorrichtung 1 über eine Entlastungsleitung 20 zugeführt. Die Entlastungsleitung 20 führt entsprechend sowohl das Entlastungsgas als auch den vom Entlastungsgas mitgerissenen Füllproduktschaum.

Die Vorrichtung 1 umfasst einen geschlossenen Abscheidebehälter 2, in welchen aus dem Ventilknoten 130 über die Entlastungsleitung 20 ein Gemisch aus Entlastungsgas, also CO₂, und Füllproduktschaum eingebracht wird, wenn über die jeweilige Entlastung des Kopfraums eines befüllten Behälters entsprechend das Entlastungsgas kontrolliert über den Ventilknoten 130 abgelassen wird und dabei Füllproduktschaum mitgerissen wird.

Der Abscheidebehälter 2 ist bevorzugt im Wesentlichen zylinderförmig oder konisch ausgebildet und die Entlastungsleitung 20 mündet in den geschlossenen Abscheidebehälter 2 bevorzugt in einem unteren Bereich des geschlossenen Abscheidebehälters 2 und tangential ein.

Da der geschlossene Abscheidebehälter 2 bevorzugt im Wesentlichen zylinderförmig oder konusförmig ausgebildet ist, kann bei einem tangentialen Einleiten des Gemisches aus dem Entlastungsgas und dem Füllproduktschaum über die Entlastungsleitung 20 bereits aufgrund der Zentrifugalkräfte ein Ablegen des Füllproduktschaums an der jeweiligen Innenwand des geschlossenen Abscheidebehälters 2 erreicht werden.

Über eine Absaugung 22 kann das Entlastungsgas, insbesondere das CO₂, im oberen Bereich des geschlossenen Abscheidebehälters 2 aus diesem abgezogen werden.

Durch das Bereitstellen des geschlossenen Abscheidebehälters 2 in Kombination mit der Absaugung 22 kann vermieden werden, dass sich im Bereich der Getränkeabfüllanlage 100 eine erhöhte Entlastungsgaskonzentration, insbesondere eine erhöhte CO₂-Konzentration, ausbildet, welche zu Gesundheitsschäden beziehungsweise Beeinträchtigungen der Mitarbeiter an der Getränkeabfüllanlage 100 führen könnte. Die Absaugung 22 saugt das Entlastungsgas dabei bevorzugt in einen Außenbereich, der beabstandet von der Getränkeabfüllanlage 100 liegt, ab und führt das Entlastungsgas bevorzugt durch einen Kamin oder mittels eines Gebläses über das Hallendach oder einen anderen Bereich aus dem Raum ab, in welchem die Getränkeabfüllanlage 100 angeordnet ist.

Im unteren Bereich des geschlossenen Getränkeabfüllbehälters 2 ist ein Sumpf 24 vorgesehen, der im schematisch gezeigten Ausführungsbeispiel mit einem flüssigen Medium 240 gefüllt ist. Das flüssige Medium 240 ist zu Beginn der Behandlung des Gemischs aus Entlastungsgas und Füllproduktschaum beispielsweise Frischwasser. In das Frischwasser mischen sich jedoch während des Abscheidens des Füllproduktschaums entsprechende Füllproduktkomponenten, welche über den Füllproduktschaum in den geschlossenen Abscheidebehälter 2 transportiert wurden.

An dem geschlossenen Abscheidebehälter 2 und bevorzugt dessen Sumpf 24 ist an dessen unterster Position ein Ablauf 242 vorgesehen, mittels welchem das flüssige Medium 240 aus dem geschlossenen Abscheidebehälter 2, bevorzugt dem Sumpf 24, abgezogen werden kann. Das flüssige Medium 240 wird mittels einer Pumpe 244 in eine Beregnungsdüse 3 gepumpt, mittels welcher das flüssige Medium 240 auf das im geschlossenen Abscheidebehälter 2 vorliegende Gemisch aus Entlastungsgas und Füllproduktschaum aufgeregnet wird. Durch das Beregnen des Gemischs aus Entlastungsgas und Füllproduktschaum wird der Füllproduktschaum im geschlossenen Behälter nieder gehalten und die Bläschen des Füllproduktschaums werden nach und nach aufgebrochen, so dass der Füllproduktschaum im Wesentlichen verflüssigt wird.

Über den im unteren Bereich des geschlossenen Abscheidebehälters 2 vorgesehenen Ablauf 242 und die Pumpe 244 kann das flüssige Medium 240 entsprechend auf den sich im geschlossenen Abscheidebehälter 2 ansammelnden Füllproduktschaum aufgeregnet werden. Damit findet eine Beregnung des über die Entlastungsleitung 20 zugeführten Füllproduktschaumes so statt, dass der Füllproduktschaum niedergehalten werden kann und ein übermäßiges Ansteigen des Niveaus des Füllproduktschaumes in dem geschlossenen Abscheidebehälter 2 vermieden werden kann, derart, dass ein Ansaugen des Füllproduktschaumes durch die Absaugung 22 für das Entlastungsgas vermieden werden kann.

Weiterhin wird über die Beregnung des Füllproduktschaums mittels der Beregnungsdüse 3 erreicht, dass der Füllproduktschaum zerschlagen wird und entsprechend nur die mittels des Füllproduktschaumes transportieren Füllproduktkomponenten in flüssiger beziehungsweise Partikelform vorliegen und im Sumpf 24 gesammelt werden.

Mittels einer Frischwasserzufuhr 246 kann die Beregnungsdüse 3 auch mit Frischwasser beschickt werden. Dies wird insbesondere dann durchgeführt, wenn der Sumpf 24 erneut mit Medium 240 befüllt werden soll, also jeweils beispielsweise zu Beginn des jeweiligen Behandlungsverfahrens.

Um das Verfahren zur Behandlung eines Gemischs aus Entlastungsgas und Füllproduktschaum durchzuführen, wird entsprechend über die Entlastungsleitung 20 ein Gemisch aus dem Füllproduktschaum und dem Entlastungsgas in den geschlossenen Abscheidebehälter 2 eingebracht. Gleichzeitig wird mittels der Beregnungsdüse 3 ein flüssiges Medium 240 auf den sich in dem geschlossenen Abscheidebehälter 2 sammelnden Füllproduktschaum aufgebracht, um den Schaum entsprechend niederzuhalten beziehungsweise zu zerschlagen. Das über die Beregnungsdüse 3 aufgebrachte flüssige Medium 240 sowie die mittels des flüssigen Mediums 240 mitgerissenen Bestandteile beziehungsweise die sich verflüssigenden Bestandteile des Füllproduktschaums fließen in den Sumpf 24. Das Entlastungsgas hingegen wird über die Absaugung 22 aus dem geschlossenen Abscheidebehälter 2 abgezogen.

Um ein Ansaugen beziehungsweise Absaugen des Füllproduktschaums über die Absaugung 22 zu vermeiden, steht die Absaugung 22 in Fluidkommunikation mit dem geschlossenen Abscheidebehälter 2 und die Absaugung 22 ist oberhalb der Beregnungsdüse 3 aus dem geschlossenen Abscheidebehälter 2 geführt.

Wenn der Spiegel des flüssigen Mediums 240 im Sumpf 24 ein bestimmtes Niveau erreicht hat, welches beispielsweise über eine obere Niveausonde 30 im geschlossenen Abscheidebehälter 2 gemessen wird, so wird durch die Betätigung der entsprechenden Ventile über die Pumpe 244 der Sumpf und insbesondere das flüssige Medium 240 im Sumpf an eine Abwasserleitung 248 ausgegeben. Die Pumpe 244 sowie die entsprechenden Ventile zur Übergabe des Mediums 240 aus dem Sumpf 24 an die Abwasserleitung 248 werden so lange geöffnet gehalten beziehungsweise betätigt, bis eine untere Niveausonde 32 ermittelt, dass im Sumpf 24 ein Spiegel des flüssigen Mediums 240 erreicht ist, welcher bei oder unterhalb eines unteren vorgegebenen Niveaus liegt. Dann werden die vorher geöffneten Ventile wieder geschlossen und das Ventil zur Beregnungsdüse 3 geöffnet, so dass das flüssige Medium 240 dann wieder aus dem Sumpf 24 die Beregnungsdüse 3 gepumpt wird und entsprechend so umgewälzt wird, dass das sich im geschlossenen Abscheidebehälter 2 angesammelte Gemisch aus Entlastungsgas und Füllproduktschaum wieder mit dem flüssigen Medium 240 beregnet wird.

Nach Produktionsende kann der Sumpf 24 beispielsweise durch Öffnung eines entsprechenden Ventils vollständig in einen Gully 25 geleert werden.

Damit ergibt sich ein effizientes Verfahren zum Behandeln eines Gemischs aus Entlastungsgas und Füllproduktschaum in einer Getränkeabfüllanlage 100.

Um nach Abschluss der jeweiligen Produktion eine Reinigung und Sterilisierung der Getränkeabfüllanlage 100 durchzuführen, wird mittels eines geschlossenen Reinigungskreislaufs eine Reinigung durchgeführt. Hierzu wird über den Füllproduktzulauf 132 in bekannter Weise dem Ventilknoten 130 ein Reinigungs- oder Sterilisationsmedium als so genanntes CIP-Medium zugeführt, welches dann zur Reinigung oder Sterilisierung des eigentlichen Füllventils über den Füllproduktmediendrehverteilerkanal 120 durch das Füllventil strömt. Aus dem Füllventil wird das CIP-Medium dann über einen CIP-Rücklauf 128 wieder dem Ventilknoten 130 zugeführt.

Mittels eines CIP-Vorlaufs 40 kann das CIP-Medium von dem Füllventilknoten 130 auch an eine CIP-Düse 42, welche sich im oberen Bereich des geschlossenen Abscheidebehälters 2 befindet, geleitet werden. Die CIP-Düse 42 ist bevorzugt als 360° Düse ausgebildet, mittels welcher die gesamte Innenwand des geschlossenen Abscheidebehälters 2 mit Reinigungs- oder Sterilisationsmedium beaufschlagt werden kann. Auf diese Weise kann der geschlossene Abscheidebehälter 2 durch das Beaufschlagen mit dem CIP-Medium vollständig gereinigt werden. Über den Ablauf 242 und die Pumpe 244 und eine entsprechende Schaltung der Ventile das CIP-Mediums wird das CIP-Medium über eine CIP-Rückleitung 44 dem Ventilknoten 130 wieder zugeführt. Entsprechend findet eine Reinigung im Kreislauf statt, wobei kein Medium austritt, sondern das Reinigungsmedium durch den geschlossenen Abscheidebehälter 2 im Kreislauf geführt wird.

In einer Alternative beziehungsweise Weiterbildung kann das CIP-Medium auch über die Entlastungsleitung 20 aus dem Ventilknoten 130 in den geschlossenen Abscheidebehälter 2 strömen, um auf diese Weise auch eine Reinigung der Entlastungsleitung 2 zu ermöglichen.

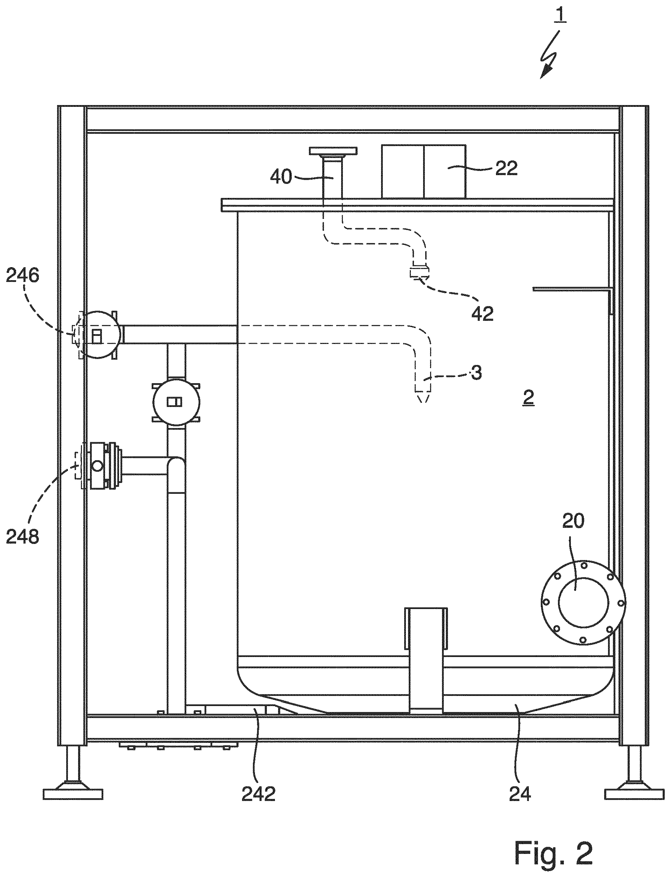

In Figur 2 ist schematisch der geschlossene Abscheidebehälter 2 in einer schematischen Seitenansicht gezeigt, wobei die Beregnungsdüse 3 schattiert im Innenraum gezeigt ist und die Zuführung der Entlastungsleitung 20 ebenfalls schematisch dargestellt ist. Der Sumpf 24 im unteren Bereich des geschlossenen Abscheidebehälters 2 sowie der Ablauf 242 sind ebenfalls dargestellt. Über einen Abwasseranschluss 248 und einen Frischwasseranschluss 246 kann dem geschlossenen Abscheidebehälter 2 Frischwasser zugeführt werden und das Abwasser abgeführt werden.

Ein CIP-Vorlauf 40 ist gezeigt, genauso wie die CIP-Düse 42, welche im Innenraum des geschlossenen Abscheidebehälters 2 vorgesehen sind und welche in der oben beschriebenen Weise zur Reinigung oder Sterilisierung der Innenwand des geschlossenen Abscheidebehälters 2 dienen.

Über die im oberen Bereich des geschlossenen Abscheidebehälters angeordnete Absaugung 22 wird das Entlastungsgas aus dem geschlossenen Abscheidebehälter 2 ausgetragen.

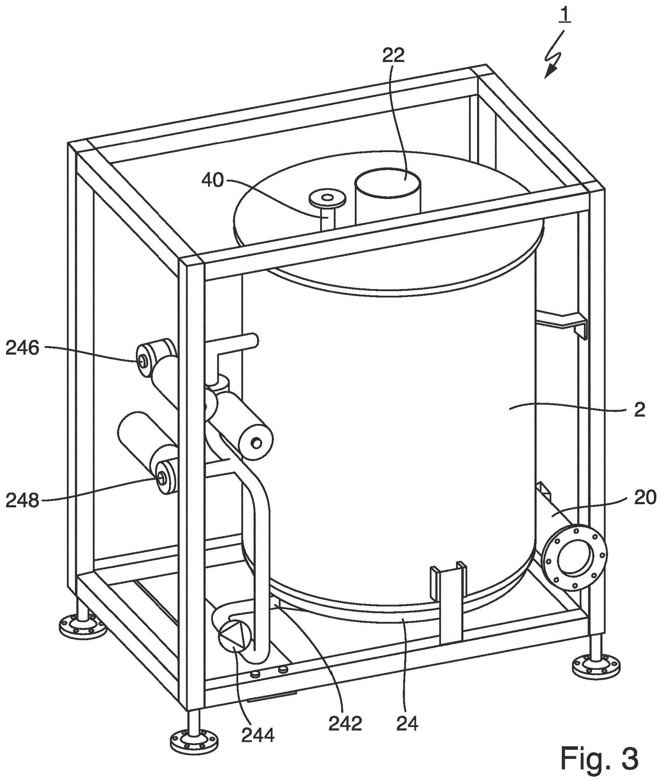

Figur 3 zeigt die Vorrichtung der Figur 2 noch einmal in einer schematischen perspektivischen Ansicht.

### Bezugszeichenliste

- 1: Vorrichtung zum Behandeln eines Gemischs aus Entlastungsgas und Füllproduktschaum
- 100: Getränkeabfüllanlage
- 110: Füllerkarussell
- 120: Füllproduktzuleitung
- 122: CO₂-Zufuhr
- 124: CO₂-Entlastung
- 126: Vakuum
- 128: CIP-Rücklauf
- 130: Ventilknoten
- 132: Füllproduktzulauf
- 134: CIP-Rücklauf
- 136: CO₂-Zufuhr
- 138: Vakuumanschluss
- 2: geschlossener Abscheidebehälter
- 20: Entlastungsleitung
- 22: Absaugung
- 24: Sumpf
- 240: Medium
- 242: Ablauf
- 244: Pumpe
- 246: Frischwasser
- 248: Abwasser
- 25: Gully
- 3: Beregnungsdüse
- 30: obere Niveausonde
- 32: untere Niveausonde
- 40: CIP-Vorlauf
- 42: CIP-Düse
- 44: CIP-Rücklauf

## Patentansprüche

1. Verfahren zum Behandeln eines Gemischs aus Entlastungsgas und Füllproduktschaum in einer Getränkeabfüllanlage (100), umfassend die Schritte:
Einführen des Gemisches aus Entlastungsgas und Füllproduktschaum in einen geschlossenen Abscheidebehälter (2) und
Absaugen des Entlastungsgases aus dem geschlossenen Abscheidebehälter (2),
**dadurch gekennzeichnet, dass**
auf das in den geschlossenen Abscheidebehälter (2) eingeführte Gemisch aus Entlastungsgas und Füllproduktschaum ein flüssiges Medium (240) zur Beregnung aufgebracht wird.

2. Verfahren gemäß Anspruch 1, **dadurch gekennzeichnet, dass** zur Beregnung des Gemischs aus Entlastungsgas und Füllproduktschaum das flüssige Medium (240) aus dem geschlossenen Abscheidebehälter (2), bevorzugt aus einem unterem Bereich des geschlossenen Abscheidebehälters (2), entnommen wird und das flüssige Medium (240) bevorzugt in einem Kreislauf geführt wird.

3. Verfahren gemäß Anspruch 1 oder 2, **dadurch gekennzeichnet, dass** im unteren Bereich, bevorzugt in einem Sumpf (24), des geschlossenen Abscheidebehälters (2) ein flüssiges Medium (240) bereitgestellt wird, welches bevorzugt zur Beregnung des Gemischs aus Entlastungsgas und Füllproduktschaum gemäß Anspruch 1 oder 2 entnommen wird.

4. Verfahren gemäß einem der vorstehenden Ansprüche, **dadurch gekennzeichnet, dass** im geschlossenen Abscheidebehälter (2) das Entlastungsgas oberhalb der Beregnung abgesaugt wird.

5. Verfahren gemäß einem der vorstehenden Ansprüche, **dadurch gekennzeichnet, dass** das flüssige Medium (240) mit dem durch die Beregnung verflüssigten Füllproduktschaum aus dem geschlossenen Abscheidebehälter (2) kontinuierlich, periodisch oder bedarfsweise abgeführt wird.

6. Verfahren gemäß einem der vorstehenden Ansprüche, **dadurch gekennzeichnet, dass** das Gemisch aus Entlastungsgas und Füllproduktschaum dem geschlossenen Abscheidebehälter (2) tangential zugeführt wird.

7. Verfahren gemäß einem der vorstehenden Ansprüche, **dadurch gekennzeichnet, dass** zur Reinigung ein Reinigungs- und/oder Sterilisationsmedium in den geschlossenen Abscheidebehälter (2) eingebracht und aus diesem abgeleitet wird, wobei das Reinigungs- und/oder Sterilisationsmedium bevorzugt auf die Innenwand des geschlossenen Abscheidebehälters (2) aufgesprüht wird und im unteren Bereich des geschlossenen Abscheidebehälters (2) ableitet wird.

8. Vorrichtung zum Behandeln eines Gemischs aus Entlastungsgas und Füllproduktschaum in einer Getränkeabfüllanlage (100), umfassend einen Abscheidebehälter (2), in welchen eine das Gemisch aus Entlastungsgas und Füllproduktschaum führende Entlastungsleitung (20) einmündet, wobei aus dem Abscheidebehälter (2) über eine Absaugung (22) das Entlastungsgas abgesaugt wird, der Abscheidebehälter (2) geschlossen ist,
**dadurch gekennzeichnet, dass**
eine Beregnungsdüse (3) in dem geschlossenen Abscheidebehälter (2) vorgesehen ist, welche ein flüssiges Medium (240) auf das im geschlossenen Abscheidebehälter (2) vorliegende Gemisch aus Entlastungsgas und Füllproduktschaum aufbringt.

9. Vorrichtung gemäß Anspruch 8, **dadurch gekennzeichnet, dass** in einem unteren Bereich des geschlossenen Abscheidebehälters (2) ein Ablauf (242), bevorzugt ein an einem Sumpf (24) des geschlossenen Abscheidebehälters (2) vorgesehener Ablauf (242), vorgesehen ist und der Ablauf (242) mit der Beregnungsdüse (3) in Fluidkommunikation steht, so dass das flüssige Medium (240) bevorzugt im Kreislauf von dem unteren Bereich des geschlossenen Abscheidebehälters (2) zu der Beregnungsdüse (3) gepumpt werden kann.

10. Vorrichtung gemäß Anspruch 8 oder 9, **dadurch gekennzeichnet, dass** eine Absaugung (22) zum Absaugen des Entlastungsgases in Fluidkommunikation mit dem geschlossenen Abscheidebehälter (2) vorgesehen ist und die Absaugung (22) bevorzugt oberhalb der Beregnungsdüse (3) aus dem geschlossenen Abscheidebehälter (2) geführt ist.

11. Vorrichtung gemäß einem der Ansprüche 8 bis 10, **dadurch gekennzeichnet, dass** der geschlossene Abscheidebehälter (2) zylinderförmig und/oder konisch ausgebildet ist und die Entlastungsleitung (20) tangential in den geschlossenen Abscheidebehälter (2) eingeführt ist.

12. Vorrichtung gemäß einem der Ansprüche 8 bis 11, **dadurch gekennzeichnet, dass** in dem geschlossenen Abscheidebehälter (2) eine CIP-Düse (42) zum Beaufschlagen der Innenwand des geschlossenen Abscheidebehälters (2) vorgesehen ist und der geschlossene Abscheidebehälter (2) in einem unteren Bereich in Fluidkommunikation mit einer CIP-Rückleitung (44) steht.

## Claims

1. Method for treating a mixture of expansion gas and fill product foam in a beverage filling plant (100), comprising the steps of:
introducing the mixture of expansion gas and fill product foam into a closed separation container (2) and
extracting the expansion gas out of the closed separation container (2) by means of suction,
**characterized in that**
a fluid medium (240) is applied by sprinkling to the mixture of expansion gas and fill product foam that is introduced into the closed separation container (2).

2. Method according to claim 1, **characterized in that** in order to sprinkle the mixture of expansion gas and fill product foam, the fluid medium (240) is extracted from the closed separation container (2), preferably from a lower area of the closed separation container (2), and the fluid medium (240) is preferably recirculated.

3. Method according to claim 1 or 2, **characterized in that** in the lower area, preferably in a sump (24), of the closed separation container (2), a fluid medium (240) is provided, which is preferably extracted for sprinkling the mixture of expansion gas and fill product foam according to claim 2 or 3.

4. Method according to one of the previous claims, **characterized in that** in the closed separation container (2) the expansion gas is extracted by suction above the sprinkler.

5. Method according to one of the previous claims, **characterized in that** the fluid medium (240), together with the fill product foam that has been liquefied by sprinkling, is conveyed out of the closed separation container (2) continuously, periodically or as required.

6. Method according to one of the previous claims, **characterized in that** the mixture of expansion gas and fill product foam is introduced tangentially into the closed separation container (2).

7. Method according to one of the previous claims, **characterized in that** for the purpose of cleaning, a cleaning and/or sterilization medium is introduced into the closed separation container (2) and conducted out of it, wherein the cleaning and/or sterilization medium is preferably sprayed onto the interior wall of the closed separation container (2) and conducted out of the closed separation container (2) in its lower area.

8. Device for treating a mixture of expansion gas and fill product foam in a beverage filling plant (100), comprising a separation container (2) into which a relief line (20) conveying the mixture of expansion gas and fill product foam discharges, wherein the expansion gas is extracted by suction from the separation container (2) by means of a suction extractor (22), the separation container (2) is closed,
**characterized in that**
a sprinkler nozzle (3) is provided in the closed separation container (2), which applies a fluid medium (240) to the mixture of expansion gas and fill product foam that is present in the closed separation container (2).

9. Device according to claim 8, **characterized in that** an outlet (242) is provided in a lower area of the closed separation container (2), preferably an outlet (242) provided in a sump (24) of the closed separation container (2), and the outlet (242) is in fluid communication with the sprinkler nozzle (3), so that the fluid medium (240) can preferably be pumped in circulation from the lower area of the closed separation container (2) to the sprinkler nozzle (3).

10. Device according to claim 8 or 9, **characterized in that** a suction extractor (22) for extracting the expansion gas is provided, in fluid communication with the closed separation container (2), and the suction extractor (22) from the closed separation container (2) is preferably disposed above the sprinkler nozzle (3).

11. Device according to one of claims 8 to 10, **characterized in that** the closed separation container (2) is substantially cylindrical and/or conical in shape, and the relief line (20) is tangentially introduced into the closed separation container (2).

12. Device according to one of claims 8 to 11, **characterized in that** a CIP nozzle (42) is provided in the closed separation container (2) to impinge on the interior wall of the closed separation container (2), and a lower area of the closed separation container (2) is in fluid communication with a CIP return line (44).

## Revendications

1. Procédé permettant le traitement d'un mélange de gaz de décharge et de mousse de produit de remplissage dans une installation d'embouteillage de boissons (100), comprenant les étapes consistant à :
introduire le mélange de gaz de décharge et de mousse de produit de remplissage dans un récipient de séparation fermé (2), et
aspirer le gaz de décharge hors du récipient de séparation fermé (2),
**caractérisé en ce que**
un milieu liquide (240) est appliqué sur le mélange de gaz de décharge et de mousse de produit de remplissage introduit dans le récipient de séparation fermé (2) pour l'aspersion.

2. Procédé selon la revendication 1, **caractérisé en ce que**, pour l'aspersion du mélange de gaz de décharge et de mousse de produit de remplissage, le milieu liquide (240) est prélevé hors du récipient de séparation fermé (2), de préférence hors d'une zone inférieure du récipient de séparation fermé (2), et le milieu liquide (240) est guidé de préférence dans un circuit.

3. Procédé selon la revendication 1 ou 2, **caractérisé en ce qu'**un milieu liquide (240) est mis à disposition dans la zone inférieure, de préférence dans un fond (24), du récipient de séparation fermé (2), lequel milieu liquide est prélevé de préférence pour l'aspersion du mélange de gaz de décharge et de mousse de produit de remplissage selon la revendication 1 ou 2.

4. Procédé selon l'une des revendications précédentes, **caractérisé en ce que**, dans le récipient de séparation fermé (2), le gaz de décharge est aspiré au-dessus de l'aspersion.

5. Procédé selon l'une des revendications précédentes, **caractérisé en ce que** le milieu liquide (240) comportant la mousse de produit de remplissage liquéfiée par l'aspersion est évacué du récipient de séparation fermé (2) de manière continue, périodique ou en cas de besoin.

6. Procédé selon l'une des revendications précédentes, **caractérisé en ce que** le mélange de gaz de décharge et de mousse de produit de remplissage est amené tangentiellement au récipient de séparation fermé (2).

7. Procédé selon l'une des revendications précédentes, **caractérisé en ce que**, pour le nettoyage, un milieu de nettoyage et/ou de stérilisation est apporté dans le récipient de séparation fermé (2) et est déversé hors de celui-ci, dans lequel le milieu de nettoyage et/ou de stérilisation est de préférence pulvérisé sur la paroi intérieure du récipient de séparation fermé (2) et est déversé dans la zone inférieure du récipient de séparation fermé (2).

8. Dispositif permettant le traitement d'un mélange de gaz de décharge et de mousse de produit de remplissage dans une installation d'embouteillage de boissons (100), comprenant un récipient de séparation (2) dans lequel débouche une conduite de décharge (20) guidant le mélange de gaz de décharge et de mousse de produit de remplissage, dans lequel le gaz de décharge est aspiré hors du récipient de séparation (2) par l'intermédiaire d'une aspiration (22), le récipient de séparation (2) étant fermé,
**caractérisé en ce que**
une buse d'aspersion (3) est prévue dans le récipient de séparation fermé (2), laquelle fournit un milieu liquide (240) sur le mélange de gaz de décharge et de mousse de produit de remplissage présent dans le récipient de séparation fermé (2).

9. Dispositif selon la revendication 8, **caractérisé en ce que**, une évacuation (242), de préférence une évacuation (242) prévue au niveau d'un fond (24) du récipient de séparation fermé (2), est prévue dans une zone inférieure du récipient de séparation fermé (2), et l'évacuation (242) est en communication fluidique avec la buse d'aspersion (3) de sorte que le milieu liquide (240) peut être pompé de préférence dans un circuit depuis la zone inférieure du récipient de séparation fermé (2) vers la buse d'aspersion (3).

10. Dispositif selon la revendication 8 ou 9, **caractérisé en ce qu'**une aspiration (22) est prévue pour l'aspiration du gaz de décharge en communication fluidique avec le récipient de séparation fermé (2) et l'aspiration (22) est guidée, de préférence au-dessus de la buse d'aspersion (3), hors du récipient de séparation fermé (2).

11. Dispositif selon l'une des revendications 8 à 10, **caractérisé en ce que** le récipient de séparation fermé (2) est réalisé en forme cylindrique et/ou conique et la conduite de décharge (20) est introduite tangentiellement dans le récipient de séparation fermé (2).

12. Dispositif selon l'une des revendications 8 à 11, **caractérisé en ce qu'**une buse CIP (42) est prévue dans le récipient de séparation fermé (2) pour la sollicitation de la paroi intérieure du récipient de séparation fermé (2), et le récipient de séparation fermé (2) est en communication fluidique avec une conduite de retour CIP (44) dans une zone inférieure.
